# FASCICULE DE BREVET EUROPEEN

(11) **EP 2 344 662 B1**
(45) Date de publication et mention de la délivrance du brevet: **07.12.2016**
(21) Numéro de dépôt: 09755979.3
(22) Date de dépôt: 07.10.2009
(51) Int. Cl.: C12Q 1/04, C12Q 1/14

(54) **MILIEU REACTIONNEL POUR LES BACTERIES STAPHYLOCOCCUS AUREUS RESISTANTES A LA METICILLINE (MRSA)**
REAKTIONSMEDIUM FÜR METHICILLINRESISTENTE STAPHYLOCOCCUS AUREUS-(MRSA-)BAKTERIEN
REACTION MEDIUM FOR METHICILLIN-RESISTANT STAPHYLOCOCCUS AUREUS (MRSA) BACTERIA

(30) Priorité: 08.10.2008 FR 0856814
(43) Date de publication de la demande: 20.07.2011
(73) Titulaire: Biomérieux, 69280 Marcy L'etoile (FR)
(72) Inventeur: ORENGA, Sylvain, F-01160 Neuville sur Ain (FR); ROBICHON, Denis, F-01150 Blyes (FR); ZAMBARDI, Gilles, F-38300 Chezeneuve (FR)
(86) Numéro de dépôt international: PCT/FR2009/051908
(87) Numéro de publication internationale: WO 2010/040951

(56) Documents cités:
- WO-A-2004/063391
- WO-A-2007/096639
- FR-A- 2 881 755
- WERTHEIM H. ET AL.: "Improved detection of methicillin-resistant Staphylococcus aureus using phenyl mannitol broth containing aztreonam and ceftizoxime" J. CLIN. MICROBIOL., vol. 39, no. 7, juillet 2001 (2001-07), pages 2660-2662, XP002281097
- VELASCO D. ET AL.: "Evaluation of different methods for detecting methicillin (oxacillin) resistance in Staphylococcus aureus" J. ANTIMICR. CHEMOTHER., vol. 55, no. 3, mars 2005 (2005-03), pages 379-382, XP002519822
- JOLY-GUILLOU M.-L.: "Intérêt du E-test dans le suivi de l'antibiothérapie", REANIMATION, vol. 15, 2006, pages 237-240,

## Description

La présente invention concerne un milieu de culture pour la détection des bactéries *Staphylococcus aureus* résistantes à la Méticilline (SARM ou MRSA). L'invention concerne également l'utilisation de ce milieu, ainsi qu'une méthode pour identifier des bactéries MRSA. Les *Staphylococcus aureus* résistants à la Méticilline sont des souches de *Staphylococcus aureus,* caractérisées par leur résistance à un antibiotique, la Méticilline, et aux antibiotiques apparentés telle que l'Oxacilline. Le plus souvent cette résistance est conférée par l'expression d'un gène, *mecA*, entraînant la production d'une protéine modifiée, PLP2a (également appelée PBP 2a ou PBP 2').

Les bactéries MRSA représentent un fort pourcentage des infections nosocomiales, et sont souvent à l'origine de problèmes de santé graves et potentiellement mortels. Transmis le plus souvent de façon croisée entre les patients via le personnel soignant, les MRSA sont très contagieux et responsables d'infections endémiques très difficiles à contrôler.

Outre un traitement adapté, le dépistage des porteurs de MRSA, et l'isolement des patients colonisés constituent la méthode la plus efficace, aujourd'hui recommandée par des organismes officiels tels que la Society for Healthcare Epidemiology of America (Société Américaine pour l'Epidémiologie Hospitalière). Un dépistage précoce et systématique est donc essentiel.

La détection des MRSA peut se faire par différentes techniques.

Il est ainsi possible de détecter des MRSA par des techniques de biologie moléculaire. A ce titre, on peut citer notamment le brevet européen EP 0 887 424.

De telles méthodes restent toutefois coûteuses en test de routine, et requièrent un personnel qualifié.

Il est également possible d'utiliser des milieux de culture conventionnels pour détecter les *Staphylococcus aureus*, tel que le milieu décrit dans le brevet européen EP 1 390 524.

La détection de MRSA se fait lors d'une étape supplémentaire, par un test d'agglutination spécifique, (Slidex MRSA, bioMérieux) ou par une méthode de diffusion sur gélose en présence d'un disque Oxacilline (recommandations du Comité de l'Antibiogramme de la Société Française de Microbiologie).

Il est également possible de mettre en culture les bactéries susceptibles d'être des MRSA sur milieux gélosés en présence d'antibiotiques. De tels milieux peuvent être également chromogènes, ce qui facilite la lecture et la détection des MRSA. On peut citer notamment le milieu décrit dans le brevet européen EP 1 543 147.

Toutefois, la détection d'une activité phosphatase dans les conditions décrites étant peu spécifique, il est nécessaire de la combiner avec la détection de plusieurs autres activités enzymatiques ce qui réduit la fertilité du milieu et accroît son coût.

De plus, WO 2004/063391 décrit des méthodes de détection de microorganismes résistants à des antibiotiques (e.g. MRSA) comprenant la culture desdits microorganismes dans ou sur agar chromogène préalablement supplémenté par au moins un antibiotique β-lactamine, e.g. toute combinaison de céphalosporines de première, deuxième, troisième et quatrième génération. En particulier D1 envisage la combinaison cefoxitine-cefotaxime et mentionne également l'utilisation de carbapenemes et monobactames. X-gal et X-glu sont cités comme agents chromogènes.

Wertheim H. et al., J. Clin. Microbiol., vol. 39, no. 7, July 2001 : 2660-2662 décrit une méthode de détection de MRSA basée sur la création d'un nouveau milieu sélectif contenant du rouge phénol, du mannitol, de l'aztréoname (monobactame) et de la ceftizoxime (céphalosporine de troisième génération).

L'invention se propose de résoudre les lacunes de l'état de la technique en présentant un nouveau milieu de détection sensible, spécifique, et rapide pour isoler et identifier des *Staphylococcus aureus* résistants à la Méticilline (MRSA).

D'une manière surprenante, les inventeurs ont mis en évidence que l'utilisation de combinaison d'antibiotiques, à une concentration faible et prédeterminée, permettait d'obtenir un excellent milieu de détection pour isoler et identifier des *Staphylococcus aureus* résistants à la Méticilline (MRSA). Cet effet est d'autant plus surprenant que les antibiotiques, utilisés séparément, ne permettent nullement de différencier des MRSA aux concentrations selon l'invention.

Avant d'aller plus avant, les définitions suivantes, nullement limitatives, permettront de mieux comprendre l'invention.

Au sens de la présente invention, on entend par milieu réactionnel, un milieu comprenant tous les éléments nécessaires à la survie et/ou à la croissance de microorganismes, telles que les

### Staphylococcus aureus.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.

Le milieu réactionnel peut être solide, semi-solide ou liquide. Par milieu solide, on entend par exemple un milieu gélifié. Préférentiellement, le milieu selon l'invention est un milieu gélifié. L'agar est l'agent gélifiant traditionnel en microbiologie pour la culture des microorganismes, mais il est possible d'utiliser de la gélatine ou de l'agarose. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

Le milieu réactionnel selon l'invention peut contenir d'éventuels autres additifs comme par exemple : des peptones, un ou plusieurs facteurs de croissance, des hydrates de carbone, un ou plusieurs agents sélectifs, des solutions tampons, un ou plusieurs gélifiants... Ce milieu réactionnel peut se présenter sous forme de liquide, de gel prêt à l'emploi, c'est à dire prêt à l'ensemencement en tube, flacon, ou sur boite de Petri. Lorsque la présentation est sous forme de gel en flacon, on réalise préférentiellement une régénération (passage à 100°C) préalable du milieu avant de couler en boîte de Petri.

Préférentiellement, le milieu selon l'invention est un milieu sélectif, c'est à dire un milieu comprenant des inhibiteurs qui privilégient la croissance des bactéries *Staphylococcus aureus.* On peut citer notamment le Chlorure de Lithium (LiCI), Azide de sodium (NaN3), Colistine, Amphotéricine, Aztréonam, Colimicine, Chlorure de Sodium (NaCl), Déféroxamine, composé vibriostatique O/129.

Au sens de la présente invention, le substrat d'une activité enzymatique ou métabolique est choisi parmi tout substrat pouvant être hydrolysé en un produit qui permet la détection, directe ou indirecte d'une activité enzymatique ou d'un métabolisme, telle que notamment une activité osidase, préférentiellement une activité alpha glucosidase, estérase, préférentiellement une activité phosphatase, peptidase, préférentiellement une activité coagulase, ou métabolisme d'un hydrate de Carbone, préférentiellement le Mannitol.

Il peut s'agir d'un substrat naturel ou synthétique. Le métabolisme du substrat provoque une variation des propriétés physico-chimiques du milieu réactionnel ou des cellules d'organismes. Cette variation peut être détectée par des méthodes physico-chimiques, notamment des méthodes optiques par l'oeil de l'opérateur ou à l'aide d'instruments, spectrométriques, électriques, magnétiques, ... Préférentiellement, il s'agit d'une variation des propriétés optiques, telles qu'une modification d'absorption, de fluorescence ou de luminescence. Comme substrat chromogène, on peut citer notamment les substrats à base d'indoxyl, flavone, alizarine, acridine, phénoxazine, nitrophénol, nitroaniline, naphtol, catéchol, hydroxyquinoline, coumarine. Préférentiellement, le(s) substrat(s) utilisé(s) dans la présente invention est(sont) à base d'indoxyl.

Comme substrat fluorescent, on peut citer notamment les substrats à base d'umbelliférone ou de coumarine, à base de résorufine, phénoxazine, naphtol, naphtylamine, 2'-hydroxyphényl-hétérocycle ou 2'-aminophényl-hétérocycle ou encore à base de fluorescéïne.

Comme substrat d'activité enzymatique alpha glucosidase, on peut citer plus particulièrement les substrats 5-Bromo-6-chloro-3-indoxyl-alpha-glucoside ; Dihydroxyflavone-alpha-glucoside ; 3,4-Cyclohexénoesculétine-alpha-glucoside ; 8-Hydroxiquinoline-alpha-glucoside ; 5-Bromo-4-chloro-3-indoxyl-alpha-glucoside ; 5-Bromo-4-chloro-3-indoxyl-N-méthyl-alpha-glucoside ; 6-Chloro-3-indoxyl-alpha-glucoside ; 5-Bromo-3-indoxyl-alpha-glucoside ; 5-Iodo-3-indoxyl-alpha-glucoside ; 6-Fluoro-3-indoxyl-alpha-glucoside ; Alizarine-alpha-glucoside ; Nitrophényl-alpha-glucoside ; 4-Méthylumbelliferyl-alpha-glucoside ; Naphtholbenzein-alpha-glucoside ; Indoxyl-N-méthyl-alpha-glucoside ; Naphtyl-alpha-glucoside ; Aminophényl-alpha-glucoside ; Dichloroaminophényl-alpha-glucoside.

Préférentiellement, le substrat utilisé dans la présente invention est le 5-Bromo-4-chloro-3-indoxyl-alpha-glucoside, préférentiellement en combinaison avec le 5-Bromo-4-chloro-3-indoxyl-N-méthyl-alpha-glucoside.

Comme substrat d'activité enzymatique phosphatase, on peut citer plus particulièrement les substrats 5-Bromo-6-chloro-3-indoxyl-phosphate ; 3,4-Cyclohexénoesculétine-phosphate ; 5-Bromo-4-chloro-3-indoxyl-phosphate ; 5-Bromo-4-chloro-3-indoxyl-N-méthyl-phosphate ; 6-Chloro-3-indoxyl-phosphate ; 5-Bromo-3-indoxyl-phosphate ; 5-Iodo-3-indoxyl-phosphate ; 6-Fluoro-3-indoxyl-phosphate ; Nitrophényl-phosphate ; 4-Méthylumbelliferyl-phosphate , Indoxyl-N-méthyl-phosphate ; Naphtyl-phosphate.

Comme substrat de coagulase, on peut citer plus particulièrement les substrats Boc-Val-Pro-Arg-7-amido-4-methylcoumarine, Bz-Phe-Val-Arg-p-nitroanilide, Z-Gly-Pro-Arg-4-Methoxy-beta-naphtylamide, Plasminogène. En général ces substrats sont utilisés en combinaison avec une source de Prothrombine telle que la Prothrombine purifiée ou le Plasma sanguin.

Le substrat utilisé dans la présente invention peut être en combinaison avec d'autres substrats, tels qu'un substrat d'osidase et notamment beta-glucosidase ou beta-ribosidase, estérase et notamment phosphatase ou phospholipase, peptidase et notamment coagulase.

Les substrats de l'invention sont utilisables dans une large gamme de pH, notamment entre pH 5,5 et 10. préférentiellement entre 6,5 et 10. Lorsque le milieu selon l'invention comprend un substrat ou plusieurs substrats d'activité enzymatique alpha glucosidase, la concentration en substrat(s) est préférentiellement comprise entre 0,01 et 2 g/l, encore plus préférentiellement entre 0,02 et 0,2 g/l, et, avantageusement, elle est de 0,1 g/l. En effet, à cette concentration de substrat, on obtient un meilleur contraste de coloration.

Le substrat peut être également un substrat métabolique, telle qu'une source de carbone, couplée à un indicateur produisant une coloration en présence de l'un des produits du métabolisme. Dans le cas du métabolisme d'un hydrate de Carbone, c'est préférentiellement le Mannitol couplé à un indicateur de pH.

Au sens de la présente invention, un antibiotique qui appartient à la famille des céphalosporines est un antibiotique préférentiellement choisi parmi
o Une céphalosporine de première génération, telle que : Cefalexine, Cefaloridine, Cefalotine, Cefazoline, Cefadroxil, Cefazedone, Cefatrizine, Cefapirine, Cefradine, Cefacetrile, Cefrodaxine, Ceftezole
o Une Céphalosporine de deuxième génération, telle que : Cefoxitine, Cefuroxime, Cefamandole, Cefaclor, Cefotetan, Cefonicide, Cefotiam, Loracarbef, Cefmetazole, Cefprozil, Ceforanide
o Une céphalosporines de troisième génération, telle que : Cefotaxime, Ceftazidime, Cefsulodine, Ceftriaxone, Cefmenoxime, Latamoxef, Ceftizoxime, Cefixime, Cefodizime, Cefetamet, Cefpiramide, Cefoperazone, Cefpodoxime, Ceftibuten, Cefdinir, Cefditoren, Ceftriaxone, Cefoperazone, Cefbuperazone
o Une céphalosporine de quatrième génération, telle que Cefepime, Cefpirome

Les céphamycines telles que Cefoxitine, Cefotetan, Cefmetazole, Cefbuperazone, Latamoxef sont une sous-famille des céphalosporines.

Dans le cadre de la présente invention, l'antibiotique qui appartient à la famille des céphalosporines est préférentiellement la Cefoxitine, et peut être en combinaison avec la Cefotaxime.

Au sens de la présente invention, un antibiotique qui appartient à la famille des carbapenemes est un antibiotique préférentiellement choisi parmi Meropeneme, Ertapeneme, Imipeneme, Doripeneme, Faropeneme.

Dans le cadre de la présente invention, l'antibiotique qui appartient à la famille des carbapenems est préférentiellement l'Ertapeneme.

Au sens de la présente invention, un antibiotique qui appartient à la famille des aminosides est un antibiotique préférentiellement choisi parmi Amikacine, Gentamicine, Isepamicine, Kanamycine, Netilmicine, Streptomycine, Tobramycine.

Par concentration infra-inhibitrice, on entend une concentration inférieure à la concentration en antibiotique nécessaire à l'inhibition des MSSA, en milieu de culture apte a la recherche de *S. aureus* à partir d'un échantillon biologique, tel que le milieu chromID™ *S. aureus* (bioMerieux). Cette concentration est inférieure à environ 3 mg/l dans le cas de la Cefoxitine, environ 2 mg/l dans le cas de Cefotaxime, environ 1 mg/l dans le cas de l'Ertapenem, environ 2 mg/l dans le cas de la Cefoperazone, environ 2 mg/l dans le cas de Cefpodoxime, environ 1 mg/l dans le cas du Cefdinir.

Par association prédéterminée de deux antibiotiques, on entend une association de deux antibiotiques particuliers, chacun des 2 étant à une concentration infra-inhibitrice particulière. Une telle association peut être déterminée notamment par le test de l'exemple A.

Par échantillon biologique, on entend un échantillon clinique, issu d'un prélèvement d'aspiration bronchique, trachéale ou pulmonaire, de liquide pleural, d'un lavage broncho-alvéolaire, d'expectorations, du sang ou d'une biopsie pulmonaire, de liquide articulaire ou péricardique ; liquide biologique ou un échantillon alimentaire, issu de tout type d'aliment. Cet échantillon peut être ainsi liquide ou solide et on peut citer d'une manière non limitative, un échantillon clinique de sang, de plasma, d'urines, de fécès, de prélèvements de nez, de périnée, de gorges, de peaux, de plaies, de liquide céphalo-rachidien, un échantillon alimentaire.

A ce titre, l'invention concerne un milieu réactionnel pour la détection et/ou l'identification de bactéries *Staphylococcus aureus* résistantes à la Méticilline (MRSA) comprenant une association prédéterminée de deux antibiotiques, un premier antibiotique qui appartient à la famille des céphalosporines et un deuxième antibiotique, ledit premier et deuxième antibiotique étant chacun à une concentration infra-inhibitrice.

Selon un mode préféré de réalisation de l'invention, ledit premier antibiotique appartient à la sous-famille des céphamycines. Selon un mode encore plus préféré de réalisation de l'invention, ledit premier antibiotique est la Cefoxitine.

Selon un mode préféré de réalisation de l'invention, ledit deuxième antibiotique appartient à la famille des carbapenems.

Selon un mode préféré de réalisation de l'invention, ledit deuxième antibiotique appartient à la famille des céphalosporines.

Selon un mode préféré de réalisation de l'invention, ledit deuxième antibiotique appartient à la famille des aminosides.

Selon un mode préféré de réalisation de l'invention, le milieu comprend en outre un substrat permettant la détection d'une activité enzymatique, préférentiellement une activité osidase, estérase ou peptidase, ou métabolique, préférentiellement le métabolisme d'un hydrate de Carbone.

Dans le cas d'une activité osidase, c'est préférentiellement une activité alpha-glucosidase. Dans le cas d'une activité estérase, c'est préférentiellement une activité phosphatase. Dans le cas d'une activité peptidase, c'est préférentiellement une activité coagulase. Dans le cas du métabolisme d'un hydrate de Carbone c'est préférentiellement le Mannitol couplé à un indicateur de pH.

Selon un mode préféré de réalisation de l'invention, le substrat est un substrat permettant la détection d'une activité enzymatique osidase, préférentiellement alpha-glucosidase.

Ledit substrat d'une activité enzymatique alpha-glucosidase est préférentiellement un indoxyl-alpha-glucoside. Préférentiellement, le substrat utilisé est le 5-Bromo-4-chloro-3-indoxyl-N-methyl-alpha-glucoside (X-N-méthyl-alpha-glucoside). Préférentiellement, ce substrat est présent dans le milieu à une concentration comprise entre 0,01 et 2 g/l, préférentiellement entre 0,02 et 0,3 g/l.

Selon un mode particulier de réalisation de l'invention, ledit milieu comprend un deuxième substrat enzymatique ou métabolique. Ce substrat peut être un substrat d'alpha-glucosidase ou un autre substrat. Préférentiellement, ce deuxième substrat est un substrat d'alpha glucosidase. Lorsque ledit milieu comprend un deuxième substrat d'alpha-glucosidase, ce substrat est préférentiellement le 5-Bromo-4-chloro-3-indoxyl-alpha-glucoside (X-alpha-glucoside). Préférentiellement, ce deuxième substrat est présent dans le milieu à une concentration comprise entre 0,01 et 2 g/l, préférentiellement entre 0,02 et 0,3 g/l.

Selon un mode préféré de réalisation de l'invention, ledit premier et/ou deuxième antibiotique appartenant à la famille des céphalosporines est choisi parmi
o une céphalosporine de première génération, préférentiellement Cefalexine, Cefaloridine, Cefalotine, Cefazoline, Cefadroxil, Cefazedone, Cefatrizine, Cefapirine, Cefradine, Cefacetrile, Cefrodaxine, Ceftezole
o une céphalosporine de deuxième génération, préférentiellement Cefoxitine, Cefuroxime, Cefamandole, Cefaclor, Cefotetan, Cefonicide, Cefotiam, Loracarbef, Cefmetazole, Cefprozil, Ceforanide
o une céphalosporine de troisième génération, préférentiellement: Cefotaxime, Ceftazidime, Cefsulodine, Ceftriaxone, Cefmenoxime, Latamoxef, Ceftizoxime, Cefixime, Cefodizime, Cefetamet, Cefpiramide, Cefoperazone, Cefpodoxime, Ceftibuten, Cefdinir, Cefditoren, Ceftriaxone, Cefoperazone, Cefbuperazone
o une céphalosporine de quatrième génération, préférentiellement Cefepime, Cefpirome

Selon un mode préféré de réalisation de l'invention, ledit deuxième antibiotique appartenant à la famille des carbapenems est choisi parmi Meropeneme, Ertapeneme, Imipeneme.

Selon un mode préféré de réalisation de l'invention, la combinaison de deux antibiotiques est choisie parmi les combinaisons Cefoxitine - Cefotaxime ou Cefoxitine - Ertapenem.

Selon un mode préféré de réalisation de l'invention, le milieu peut comprendre, en outre, au moins un inhibiteur qui privilégie la croissance des bactéries *Staphylococcus aureus,* tel que Chlorure de lithium(LiCI), Azide de sodium (NaN3), Colistine, Amphotéricine, Aztréonam, Colimicine, Chlorure de sodium(NaCl) et Déféroxamine.

Selon un mode préféré de réalisation de l'invention, le milieu comprend, en outre, un mélange d'inhibiteurs, comprenant quatre inhibiteurs, qui privilégie la croissance des bactéries du genre *Staphylococcus*, qui sont LiCl, composé vibriostatique O/129, Aztréonam, et. Amphotéricine. Selon un mode préféré de réalisation de l'invention, l'association de deux antibiotiques comprend la Cefoxitine et la Cefotaxime. Préférentiellement, ladite concentration infra inhibitrice en Cefoxitine est comprise entre 0,25 et 1,5 mg/l, préférentiellement entre 0,5 et 1 mg/l. Préférentiellement, ladite concentration infra inhibitrice en Cefotaxime est comprise entre 0,25 et 1,5 mg/l, préférentiellement entre 0,5 et 1 mg/l.

Selon un mode préféré de réalisation de l'invention, l'association de deux antibiotiques comprend la Cefoxitine et la Ceftriaxone. Préférentiellement, ladite concentration infra inhibitrice en Cefoxitine est comprise entre 0,25 et 1,5 mg/l, préférentiellement entre 0,5 et 1 mg/l. Préférentiellement, ladite concentration infra inhibitrice en Ceftriaxone est comprise entre 0,25 et 1,5 mg/l, préférentiellement entre 0,5 et 1 mg/l.

Selon un mode préféré de réalisation de l'invention, l'association de deux antibiotiques comprend la Cefoxitine et l'Ertapenem. Préférentiellement, ladite concentration infra inhibitrice en Cefoxitine est comprise entre 0,25 et 1,5 mg/l, préférentiellement entre 0,5 et 1 mg/l. Préférentiellement, ladite concentration infra inhibitrice en Ertapenem est comprise entre 0,5 et 0,75 mg/l.

Selon un mode préféré de réalisation de l'invention, l'association de deux antibiotiques comprend la Cefoxitine et la Cefpodoxime. Préférentiellement, ladite concentration infra inhibitrice en Cefoxitine est comprise entre 0,25 et 1,5 mg/l, préférentiellement entre 0,5 et 1 mg/. Préférentiellement, ladite concentration infra inhibitrice en Cefpodoxime est comprise entre 0,75 et 1 mg/l, préférentiellement entre 0,5 et 1 mg/l.

Selon un mode préféré de réalisation de l'invention, l'association de deux antibiotiques comprend la Cefoxitine et la Cefoperazone. Préférentiellement, ladite concentration infra inhibitrice en Cefoxitine est comprise entre 0,25 et 1,5 mg/l, préférentiellement entre 0,5 et 1 mg/. Préférentiellement, ladite concentration infra inhibitrice en Cefoperazone est comprise entre 0,5 et 0,75 mg/l, préférentiellement entre 0,75 et 1 mg/l.

Selon un mode préféré de réalisation de l'invention, l'association de deux antibiotiques comprend la Cefoxitine et le Cefdinir. Préférentiellement, ladite concentration infra inhibitrice en Cefoxitine est comprise entre 0,25 et 1,5 mg/l, préférentiellement entre 0,25 et 0,75 mg/l. Préférentiellement, ladite concentration infra inhibitrice en Cefdinir est comprise entre 0,05 et 0,5 mg/l, préférentiellement entre 0,1 et 0,25 mg/l.

L'invention concerne également l'utilisation *in vitro* d'un milieu réactionnel tel que défini ci avant pour isoler et identifier des bactéries *Staphylococcus aureus* résistantes à la Méticilline (MRSA).

Lors de l'utilisation de ce milieu, les MRSA sont préférentiellement détectés par une activité α-glucosidase spécifique qui permet d'obtenir des colonies colorées ou fluorescentes. Les autres espèces de *Staphylococcus aureus* apparaissent incolores ou d'une couleur ou fluorescence différente de celle des colonies de *S. aureus.*

L'invention concerne enfin un procédé de détection et/ou d'identification de bactéries *Staphylococcus aureus* résistantes à la Méticilline (MRSA) dans un échantillon biologique selon lequel
a) on ensemence l'échantillon biologique susceptible de contenir des bactéries *Staphylococcus aureus* résistantes à la Méticilline (MRSA) sur un milieu réactionnel tel que défini ci avant
b) on incube
c) on identifie les colonies de MRSA.

L'incubation est préférentiellement réalisée à une température comprise entre 30°C et 42°C. Les MRSA sont préférentiellement détectées par une activité α-glucosidase spécifique qui permet d'obtenir des colonies colorées ou fluorescentes. Les autres espèces de *Staphylococcus* apparaissent incolores ou d'une couleur ou fluorescence différente de celle des colonies de *S. aureus.*

Les exemples suivants sont donnés à titre illustratif et n'ont aucun caractère limitatif. Ils permettront de mieux comprendre l'invention.

### Exemple A - Test pour déterminer l'association prédéterminée de deux antibiotiques selon l'invention

Le test ci dessous peut être mis en oeuvre pour définir l'association prédéterminée de deux antibiotiques selon l'invention, qui dépend des antibiotiques employés et plus généralement de la formulation du milieu réactionnel.

Pour aider à sa compréhension, ce test est mis en oeuvre ci-dessous dans le cas d'une combinaison Cefoxitine et Cefotaxime, à partir d'un kit de souches de microorganismes, comprenant des MSSA et des MRSA, mais ce test peut bien évidemment être mis en oeuvre pour d'autres antibiotiques.

Dix milieux réactionnels aptes à la recherche de *S aureus* dans un échantillon biologique (tel qu'un milieu chromID™ S. aureus, bioMérieux) et différentes concentrations en Cefoxitine (entre 0 et 3 mg/l) et Cefoxatime (entre 0 et 2 mg/) sont utilisés pour obtenir des concentrations en antibiotiques comprises entre 0 et 5 mg/l. Les différentes concentrations sont espacées de façon régulière, par exemple selon une distribution arithmétique ou géométrique. Chacun des milieux est aliquoté de façon à ce que chaque souche de microorganisme puisse être ensemencée en culture pure sur chacun des milieux. Après un temps d'incubation adapté, préférentiellement 18 à 24 heures, à une température appropriée, préférentiellement 30 à 37°C, les milieux sont examinés de façon à sélectionner le milieu comprenant une association en Cefoxitine et Cefotaxime permettant de révéler le plus grand nombre de MRSA tout en les différenciant du plus grand nombre de souches MSSA.

Il peut être nécessaire de répéter l'expérimentation en adaptant les concentrations en chacun des antibiotiques.

### Exemple B - Milieu selon l'invention comprenant un substrat alpha glucosidase

### 1. Préparation du milieu selon l'invention

Les milieux testés dans les expériences ci après étaient des milieux comprenant comme milieu de base le milieu chromID MRSA (bioMérieux réf. 43 451), et comprenant les éléments suivants
**Milieu T** : milieu témoin chromID MRSA (réf. 43 451), comprenant notamment un substrat X-N-méthyl-alpha-glucoside à une concentration de 0,1 g/l et de la Cefoxitine à 4 mg/l.
**Milieu S** : milieu T, comprenant en outre, un substrat X-alpha-Glucoside, à une concentration de 25, 37, 45 ou 50 mg/l
**Milieu A** : milieu S (concentration X-alpha-glucoside : 45 mg/l la Cefoxitine étant substituée par le Ceftriaxone, à une concentration de 1 ; 2 ; 4 ; 8 ; 16 ; 32 mg/l
**Milieu B** : milieu S (concentration X-alpha-glucoside : 45 mg/l la Cefoxitine étant substituée par le Cefotaxime à une concentration de 1 ; 2 ; 4 ; 8 ; 16 ; 32 mg/l
**Milieu C** : milieu S (concentration X-alpha-glucoside : 45 mg/l), la Cefoxitine étant substituée par l'Ertapenem à une concentration de 0,1 ; 0,25 ; 0,5 ; 0,75 ; 1 mg/l
**Milieu D** : milieu S (concentration X-alpha-glucoside : 45 mg/l), la Cefoxitine étant substituée par le Cefoperazone à une concentration de 0,5 ; 1 ; 1,5 ; 2 mg/l
**Milieu E** : milieu S (concentration X-alpha-glucoside : 45 mg/l), la Cefoxitine étant substituée par le Cefpodoxime à une concentration de 0,5 ; 1 ; 1,5 ; 2 mg/l
**Milieu F** : milieu S (concentration X-alpha-glucoside : 45 mg/l), la Cefoxitine étant substituée par une combinaison Cefoxitine/Cefotaxime aux concentrations ci dessous

| | | | | |
|---|---|---|---|---|
| **[Cefoxitine] en mg/l** | 0,5 | 0,5 | 1 | 1 |
| **[Cefotaxime] en mg/l** | 0,5 | 1 | 0,5 | 1 |

**Milieu G** : milieu S (concentration X-alpha-glucoside : 45 mg/l), la Cefoxitine étant substituée par une combinaison Cefoxitine/Ceftriaxone aux concentrations ci dessous

| | | | | |
|---|---|---|---|---|
| **[Cefoxitine] en mg/l** | 0,5 | 0,5 | 1 | 1 |
| **[Ceftriaxone] en mg/l** | 0,5 | 1 | 0,5 | 1 |

**Milieu H** : milieu S (concentration X-alpha-glucoside : 45 mg/l), la Cefoxitine étant substituée par une combinaison Cefoxitine/Ertapenem aux concentrations ci dessous

| | | | | | | | | |
|---|---|---|---|---|---|---|---|---|
| **[Cefoxitine] en mg/l** | 0,5 | 0,5 | 0,5 | 0,5 | 0,75 | 0,75 | 0,75 | 0,75 |
| **[Ertapenem] en mg/l** | 0,25 | 0,5 | 0,75 | 1 | 0,25 | 0,5 | 0,75 | 1 |

**Milieu I** : milieu S (concentration X-alpha-glucoside : 45 mg/l), la Cefoxitine étant substituée par une combinaison Cefoxitine/Cefpodoxime aux concentrations ci dessous

| | | | | | | | | |
|---|---|---|---|---|---|---|---|---|
| **[Cefoxitine] en mg/l** | 0,5 | 0,5 | 0,5 | 0,5 | 0,75 | 0,75 | 0,75 | 0,75 |
| **[Cefpodoxime] en mg/l** | 0,5 | 1 | 1,5 | 2 | 0,5 | 1 | 1,5 | 2 |

**Milieu J** : milieu S (concentration X-alpha-glucoside : 45 mg/l), la Cefoxitine étant substituée par une combinaison Cefoxitine/Cefoperazone aux concentrations ci dessous

| | | | | | | | | |
|---|---|---|---|---|---|---|---|---|
| **[Cefoxitine] en mg/l** | 0,5 | 0,5 | 0,5 | 0,5 | 0,75 | 0,75 | 0,75 | 0,75 |
| **[Cefoperazone] en mg/l** | 0,5 | 0,75 | 1 | 1,5 | 0,5 | 0,75 | 1 | 1,5 |

**Milieu K** : milieu S (concentration X-alpha-glucoside : 45 mg/l), la Cefoxitine étant substituée par une combinaison Cefoxitine/Cefotaxime, aux concentrations ci dessous, comprenant en outre un mélange d'inhibiteurs privilégiant la croissance des *Staphylococcus aureus.*

| | | | | | | |
|---|---|---|---|---|---|---|
| **[Cefoxitine] en mg/l** | 0,75 | 0,75 | 0,75 | 0,75 | 0,75 | 0,75 |
| **[Cefotaxime] en mg/l** | 0,5 | 0,55 | 0,6 | 0,65 | 0,7 | 0,75 |

**Milieu L** : milieu S (concentration X-alpha-glucoside : 45 mg/l), la Cefoxitine étant substituée par le Cefdinir à une concentration de 0,25 ; 0,5 ; 1 ; 2 ; 4 ; 8 mg/l
**Milieu M** : milieu S (concentration X-alpha-glucoside : 45 mg/l), la Cefoxitine étant substituée par une combinaison Cefoxitine/Cefdinir, aux concentrations ci dessous

| | | | | | | | | | |
|---|---|---|---|---|---|---|---|---|---|
| **[Cefoxitine] en mg/l** | 0,25 | 0,5 | 0,75 | 0,25 | 0,5 | 0,75 | 0,25 | 0,5 | 0,75 |
| **[Cefdinir] en mg/l** | 0,1 | 0,1 | 0,1 | 0,25 | 0,25 | 0,25 | 0,5 | 0,5 | 0,5 |

### 2. Ensemencement et lecture des milieux

Différents sets de souches de bactéries, toutes issues de la collection de la Demanderesse, mises en suspension dans de l'eau physiologique, ont été ensemencées pour donner des colonies isolées sur le milieu. Les boîtes ont été incubées à 37 °C pendant 48 heures. Les colonies formées ont été examinées visuellement après 18h ou 24 heures d'incubation. L'intensité de coloration était également observée selon une échelle de 0 à 4 (0 : absence de coloration, 4 : coloration très intense).

Les souches détectées correspondent aux souches formant des colonies colorées sur le milieu.

### 3. Résultats :

### 3.1 Milieu pour détecter des MRSA comprenant 2 substrats d'alpha-glucosidase

Les résultats obtenus lors de l'utilisation de un ou deux substrats d'alpha-glucosidase sont présentés dans le tableau 1.

**Tableau 1 - Intensité de coloration des colonies lors de l'utilisation de 2 substrats d'alpha glucosidase**

| | | **Milieu T** | **Milieu S [X α Glu] = 25 mg/l** | **Milieu [X α Glu] = 37 mg/l** | **Milieu [X α Glu] = 50 mg/l** |
|---|---|---|---|---|---|
| Souches | Incubation | Intensité de coloration verte | Intensité de coloration verte | Intensité de coloration verte | Intensité de coloration verte |
| MRSA | 18h | 2 | 2 | 2,5 | 3 |
| | 24h | 2 | 2 | 3 | 3 |
| | > 40h | 2 | 2,5 | 3 | 3 |
| MRSA | 18h | 0 | 0 | 0 | 0 |
| | 24h | 2 | 2 | 2,5 | 3 |
| | > 40h | 3 | 3 | 4 | 4 |
| MRSA | 18h | 1,5 | 1,5 | 2 | 3 |
| | 24h | 2 | 2 | 4 | 4 |
| | > 40h | 2 | 3 | 4 | 4 |
| MRSA | 18h | 2 | 2 | 3 | 3 |
| | 24h | 2 | 2 | 4 | 4 |
| | > 40h | 2,5 | 2,5 | 4 | 4 |
| MRSA | 18h | 0,5 | 0,5 | 0 | 0 |
| | 24h | 1 | 0,5 | 1,5 | 2,5 |
| | > 40h | 2,5 | 3 | 4 | 4 |
| MRSA | 18h | 2 | 2 | 2,5 | 3 |
| | 24h | 2,5 | 2,5 | 2,5 | 2,5 |
| | > 40h | 2,5 | 3 | 4 | 4 |
| MRSA | 18h | 0 | 0 | 0 | 0 |
| | 24h | 1 | 1 | 3 | 4 |
| | > 40h | 2 | 3 | 4 | 4 |
| MRSA | 18h | 0 | 0 | 0 | 0 |
| | 24h | 0 | 0 | 0 | 0 |
| | > 40h | 3 | 3 | 4 | 4 |
| MRSA | 18h | 1,5 | 1,5 | 3 | 2,5 |
| | 24h | 2 | 2 | 4 | 4 |
| | > 40h | 2,5 | 3 | 4 | 4 |
| MRSA | 18h | 0 | 0 | 0 | 0 |
| | 24h | 0 | 0 | 2 | 3 |
| | > 40h | 2,5 | 3 | 4 | 4 |

L'ajout d'un deuxième substrat d'alpha-glucosidase permettait d'intensifier fortement la coloration des colonies, permettant ainsi de mieux repérer les colonies de MRSA.

### 3.2 Milieu pour détecter des MRSA comprenant un antibiotique choisi parmi la Ceftriaxone, la Cefotaxime, l'Ertapenem, la Cefoperazone, la Cefpodoxime ou le Cefdinir

Les résultats obtenus lors de la substitution de la Cefoxitine par un autre antibiotique sont présentés dans les tableaux 2a à 2f.

**Tableau 2a - Substitution de la Cefoxitine par la Ceftriaxone**

| | Milieu | T | A | | | | | |
|---|---|---|---|---|---|---|---|---|
| | Antibiotique | Cefoxitine | Ceftriaxone | | | | | |
| | Concentration (mg/l) | 4 | 1 | 2 | 4 | 8 | 16 | 32 |
| MRSA (Nb souches détectées / Nb de souches) | Lecture 18h | 7/10 | 9/10 | 8/10 | 3/10 | | | |
| | Lecture 24h | 8/10 | 10/10 | 10/10 | 6/10 | 3/10 | | |
| MSSA (Nb souches détectées / Nb de souches) | Lecture 18h | 3/10 | 9/10 | 7/10 | 3/10 | | | |
| | Lecture 24h | 3/10 | 9/10 | 7/10 | 3/10 | | | |

**Tableau 2b - Substitution de la Cefoxitine par la Cefotaxime**

| | Milieu | T | B | | | | | |
|---|---|---|---|---|---|---|---|---|
| | Antibiotique | Cefoxitine | Cefotaxime | | | | | |
| | Concentration (mg/l) | 4 | 1 | 2 | 4 | 8 | 16 | 32 |
| MRSA (Nb souches détectées / Nb de souches) | Lecture 18h | 7/10 | 6/10 | 3/10 | 1/10 | | | |
| | Lecture 24h | 9/10 | 9/10 | 5/10 | 3/10 | | | |
| MSSA (Nb souches détectées / Nb de souches) | Lecture 18h | 3/10 | 7/10 | 3/10 | | | | |
| | Lecture 24h | 3/10 | 7/10 | 3/10 | | | | |

**Tableau 2c - Substitution de la Cefoxitine par l'Ertapenem**

| | Milieu | T | C | | | | |
|---|---|---|---|---|---|---|---|
| | Antibiotique | Cefoxitine | Ertapenem | | | | |
| | Concentration (mg/l) | 4 | 0,1 | 0,25 | 0,5 | 0,75 | 1 |
| MRSA (Nb souches détectées / Nb de souches) | Lecture 18h | 7/10 | 9/10 | 9/10 | 8/10 | 8/10 | 5/10 |
| | Lecture 24h | 7/10 | 10/10 | 10/10 | 10/10 | 9/10 | 9/10 |
| MSSA (Nb souches détectées / Nb de souches) | Lecture 18h | | 10/10 | 10/10 | 10/10 | 8/10 | 4/10 |
| | Lecture 24h | | 10/10 | 10/10 | 10/10 | 9/10 | 6/10 |

**Tableau 2d - Substitution de la Cefoxitine par la Cefoperazone**

| | Milieu | T | D | | | |
|---|---|---|---|---|---|---|
| | Antibiotique | Cefoxitine | Cefoperazone | | | |
| | Concentration (mg/l) | 4 | 0,5 | 1 | 1,5 | 2 |
| MRSA (Nb souches détectées / Nb de souches) | Lecture 18h | 5/10 | 8/10 | 8/10 | 5/10 | 4/10 |
| | Lecture 24h | 5/10 | 10/10 | 8/10 | 6/10 | 5/10 |
| MSSA (Nb souches détectées / Nb de souches) | Lecture 18h | | 10/10 | 9/10 | 5/10 | 4/10 |
| | Lecture 24h | | 10/10 | 9/10 | 6/10 | 4/10 |

**Tableau 2e - Substitution de la Cefoxitine par la Cefpodoxime**

| | Milieu | T | E | | | |
|---|---|---|---|---|---|---|
| | Antibiotique | Cefoxitine | Cefpodoxime | | | |
| | Concentration (mg/l) | 4 | 0,5 | 1 | 1,5 | 2 |
| MRSA (Nb souches détectées / Nb de souches) | Lecture 18h | 5/10 | 9/10 | 8/10 | 8/10 | 5/10 |
| | Lecture 24h | 5/10 | 10/10 | 9/10 | 8/10 | 7/10 |
| MSSA (Nb souches détectées / Nb de souches) | Lecture 18h | | 10/10 | 10/10 | 8/10 | 6/10 |
| | Lecture 24h | | 10/10 | 10/10 | 8/10 | 6/10 |

**Tableau 2f - Substitution de la Cefoxitine par le Cefdinir**

| | Milieu | T | L | | | | | |
|---|---|---|---|---|---|---|---|---|
| | Antibiotique | Cefoxitine | Cefdinir | | | | | |
| | (Concentration (mg/l) | 4 | 0,25 | 0,5 | 1 | 2 | 4 | 8 |
| MRSA (Nb souches détectées / Nb de souches) | Lecture 18h | 5/10 | 3/10 | 3/10 | 2/10 | _ | _ | _ |
| | Lecture 24h | 6/10 | 4/10 | 4/10 | 4/10 | 1/10 | _ | _ |
| MSSA (Nb souches détectées / Nb de souches) | Lecture 18h | _ | _ | _ | _ | _ | _ | _ |
| | Lecture 24h | _ | 5/10 | 1/10 | _ | _ | _ | |

Les antibiotiques Cefoxatime, Ceftriaxone, Ertapenem, Cefpodoxime, Cefoperazone et Cefdimir ne permettaient pas d'obtenir seuls une sensibilité et une spécificité permettant la discrimination des MRSA et des MSSA.

### 3.3 - Milieu pour détecter des MRSA comprenant une combinaison d'antibiotiques choisi parmi les couples Cefoxitine/Ceftriaxone, Cefoxitine/Cefotaxime, Cefoxitine/Ertapenem, Cefoxitine/Cefoperazone ou Cefoxitine/Cefpodoxime

Les résultats obtenus lors de l'utilisation de combinaisons d'antibiotiques sont présentés dans le tableau 3.

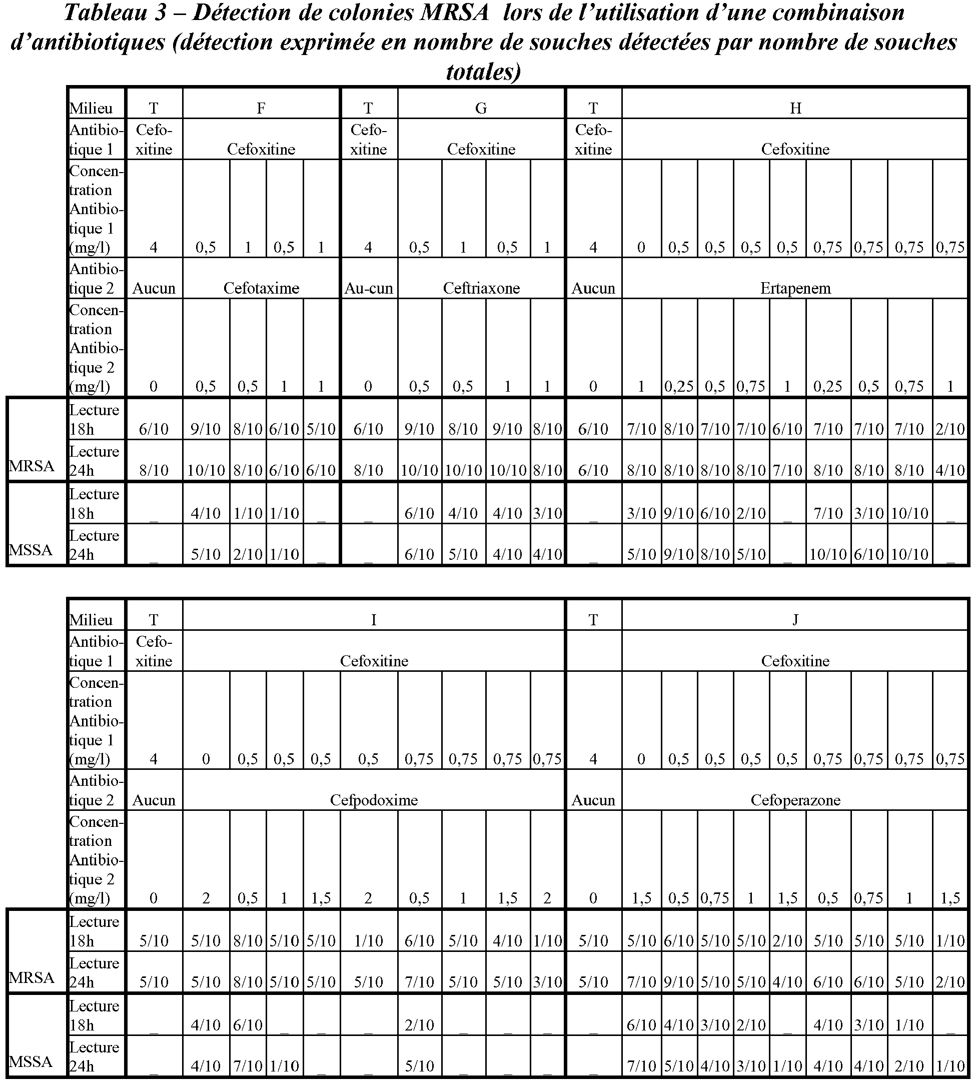

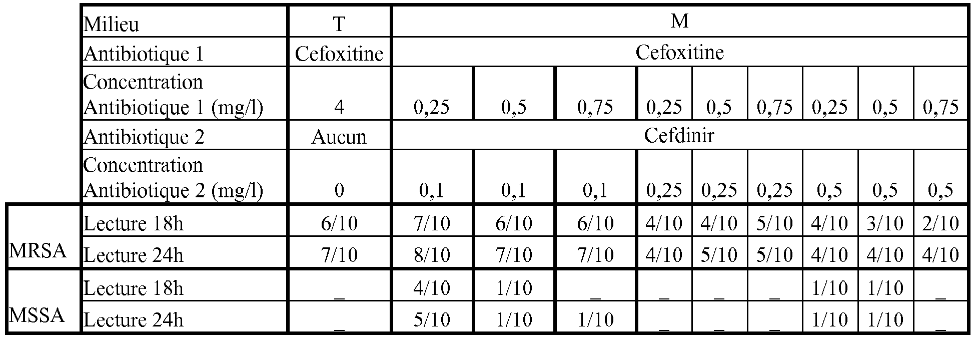

Les associations d'antibiotiques ci dessus permettaient d'obtenir une spécificité et une sensibilité supérieure à celles obtenues lors de l'utilisation d'un seul antibiotique.

### 3.4 - Milieu pour détecter des MRSA comprenant une combinaison d'antibiotiques Cefoxitine/Cefotaxime et un mélange d'inhibiteurs privilégiant la croissance des Staphylococcus aureus.

Les résultats obtenus lors de l'utilisation de combinaisons d'antibiotiques sont présentés dans le tableau 4.

**Tableau 4 - Détection de colonies MRSA lors de l'utilisation d'une combinaison d'antibiotiques Cefoxitine/Cefotaxime et un mélange d'inhibiteurs (détection exprimée en nombre de souches détectées par nombre de souches totales)**

| | Milieu | T | K | | | | | |
|---|---|---|---|---|---|---|---|---|
| | Antibiotique 1 | Cefoxitine | Cefoxitine | | | | | |
| | Concentration Antibiotique 1 (mg/l) | 4 | 0,5 | 0,55 | 0,6 | 0,65 | 0,7 | 0,75 |
| | Antibiotique 2 | Aucun | Cefotaxime | | | | | |
| | Concentration Antibiotique 2 (mg/l) | 0 | 0,75 | 0,75 | 0,75 | 0,75 | 0,75 | 0,75 |
| MRSA | Lecture 18h | 5/10 | 5/10 | 5/10 | 5/10 | 5/10 | 5/10 | 5/10 |
| | Lecture 24h | 9/10 | 8/10 | 9/10 | 8/10 | 8/10 | 8/10 | 9/10 |
| MSSA | Lecture 18h | _ | _ | _ | _ | _ | _ | _ |
| | Lecture 24h | _ | 3/10 | 2/10 | 1/10 | _ | _ | _ |

La combinaison d'antibiotiques Cefoxitine/Cefotaxime associée à un mélange d'inhibiteurs privilégiant la croissance des *Staphylococcus aureus* permettait d'obtenir une excellente spécificité et sensibilité.

### Exemple C - Milieu selon l'invention comprenant un substrat phosphatase

Les expériences similaires à celles présentées dans l'exemple B ont été réalisées, le substrat alpha glucosidase étant substitué par un substrat de phosphatase 6-Chloro-3-Indoxyl phosphate (Rose-Phosphate), l'association en antibiotiques étant cefoxitine et cefotaxime Les résultats obtenus sont présentés dans le tableau 5.

**Tableau 5 - Détection de colonies MRSA lors de l'utilisation d'un substrat de phosphatase et d'une combinaison d'antibiotiques cefoxitine / cefotaxime.**

| | Milieu | T | M | | | | | |
|---|---|---|---|---|---|---|---|---|
| | Antibiotique 1 | Cefoxitine | Cefoxitine | | | | | |
| | Concentration Antibiotique 1 (mg/l) | 4 | 0,75 | 0,75 | 0,75 | 0,75 | 1 | 1 |
| | Antibiotique 2 | Aucun | Cefotaxime | | | | | |
| | Concentration Antibiotique 2 (mg/l) | 0 | 0 | 0,5 | 0,75 | 1 | 0,5 | 0,75 |
| MRSA | Lecture 18h | 10/10 | 10/10 | 9/10 | 10/10 | 10/10 | 10/10 | 10/10 |
| | Lecture 24h | 10/10 | 10/10 | 9/10 | 10/10 | 10/10 | 10/10 | 10/10 |
| MSSA | Lecture 18h | _ | 10/10 | _ | _ | _ | _ | _ |
| | Lecture 24h | _ | 10/10 | 2/10 | _ | _ | _ | _ |

La combinaison d'antibiotiques Cefoxitine/Cefotaxime associée au substrat 6-Chloro-3-Indoxyl phosphate (Rose-Phosphate) permettait d'obtenir une excellente spécificité et sensibilité.

## Revendications

1. Milieu réactionnel pour la détection et/ou l'identification de bactéries *Staphylococcus aureus* résistantes à la Méticilline (MRSA) comprenant une association prédéterminée de deux antibiotiques, un premier antibiotique qui appartient à la famille des céphalosporines et un deuxième antibiotique, ledit premier et deuxième antibiotique étant chacun à une concentration infra-inhibitrice.

2. Milieu réactionnel selon la revendication 1 selon lequel ledit premier antibiotique appartient à la sous-famille des céphamycines.

3. Milieu réactionnel selon la revendication 1 ou 2 selon lequel ledit deuxième antibiotique appartient à la famille des carbapenems.

4. Milieu réactionnel selon la revendication 1 ou 2 selon lequel ledit deuxième antibiotique appartient à la famille des céphalosporines.

5. Milieu réactionnel selon l'une quelconque des revendications 1 à 4 selon lequel il comprend en outre un substrat permettant la détection d'une activité enzymatique ou métabolique.

6. Milieu réactionnel selon la revendication 5, selon lequel ladite activité enzymatique est une activité osidase, estérase ou peptidase.

7. Milieu réactionnel selon la revendication 6, selon lequel ladite activité enzymatique osidase est une activité alpha-glucosidase.

8. Milieu réactionnel selon la revendication 5, selon lequel ladite activité métabolique est le métabolisme d'un hydrate de Carbone.

9. Milieu réactionnel selon l'une quelconque des revendications 1 à 8 selon lequel il comprend, en outre, un deuxième substrat d'une activité enzymatique ou métabolique.

10. Milieu réactionnel selon la revendication 9 selon lequel ledit un deuxième substrat est un substrat d'une activité enzymatique alpha-glucosidase.

11. Milieu réactionnel selon l'une quelconque des revendications 1 à 10 selon lequel ledit premier et/ou deuxième antibiotique appartenant à la famille des céphalosporines est choisi parmi :
o Une céphalosporine de première génération préférentiellement choisi parmi Cefalexine, Cefaloridine, Cefalotine, Cefazoline, Cefadroxil, Cefazedone, Cefatrizine, Cefapirine, Cefradine, Cefacetrile, Cefrodaxine, Ceftezole ;
o Une Céphalosporine de deuxième génération, préférentiellement choisi parmi Cefoxitine, Cefuroxime, Cefamandole, Cefaclor, Cefotetan, Cefonicide, Cefotiam, Loracarbef, Cefmetazole, Cefprozil, Ceforanide ;
o Une céphalosporines de troisième génération, préférentiellement choisi parmi Cefotaxime, Ceftazidime, Cefsulodine, Ceftriaxone, Cefmenoxime, Latamoxef, Ceftizoxime, Cefixime, Cefodizime, Cefetamet, Cefpiramide, Cefoperazone, Cefpodoxime, Ceftibuten, Cefdinir, Cefditoren, Ceftriaxone, Cefoperazone, Cefbuperazone ;
o Une céphalosporine de quatrième génération, préférentiellement choisi parmi Cefepime, Cefpirome.

12. Milieu réactionnel selon l'une quelconque des revendications 1 à 11 selon lequel ledit deuxième antibiotique appartenant à la famille des carbapenems est choisi parmi Meropeneme, Ertapeneme, Imipeneme.

13. Milieu réactionnel selon l'une quelconque des revendications 1 à 12 selon lequel la combinaison de deux antibiotiques est choisie parmi la combinaison Cefoxitine - Cefotaxime ou Cefoxitine - Ertapenem.

14. Utilisation *in vitro* d'un milieu réactionnel selon l'une quelconque des revendications 1 à 13 pour isoler et identifier des bactéries *Staphylococcus aureus* résistantes à la Méticilline (MRSA).

15. Procédé de détection et/ou d'identification de bactéries *Staphylococcus aureus* résistantes à la Méticilline (MRSA) dans un échantillon biologique selon lequel
a) on ensemence l'échantillon biologique susceptible de contenir des bactéries *Staphylococcus aureus* résistantes à la méticilline MRSA sur un milieu réactionnel selon l'une quelconque des revendications 1 à 13
b) on incube
c) on identifie les colonies comme étant des colonies de MRSA.

## Patentansprüche

1. Reaktionsmedium für den Nachweis und/oder die Identifikation von methicillinresistenten *Staphylococcus aureus*-Bakterien (MRSA), das eine vorbestimmte Kombination von zwei Antibiotika umfasst, nämlich einem ersten Antibiotikum, das zur Familie der Cephalosporine gehört, und einem zweiten Antibiotikum, wobei das erste und das zweite Antibiotikum jeweils in einer Konzentration vorliegen, die unter der Hemmkonzentration liegt.

2. Reaktionsmedium nach Anspruch 1, wobei das erste Antibiotikum zur Unterfamilie der Cephamycine gehört.

3. Reaktionsmedium nach Anspruch 1 oder 2, wobei das zweite Antibiotikum zur Familie der Carbapeneme gehört.

4. Reaktionsmedium nach Anspruch 1 oder 2, wobei das zweite Antibiotikum zur Familie der Cephalosporine gehört.

5. Reaktionsmedium nach einem der Ansprüche 1 bis 4, das weiterhin ein Substrat umfasst, das den Nachweis einer enzymatischen oder metabolischen Aktivität gestattet.

6. Reaktionsmedium nach Anspruch 5, wobei es sich bei der enzymatischen Aktivität um eine Osidase-, Esterase- oder Peptidaseaktivität handelt.

7. Reaktionsmedium nach Anspruch 6, wobei es sich bei der enzymatischen Osidaseaktivität um eine alpha-Glucosidaseaktivität handelt.

8. Reaktionsmedium nach Anspruch 5, wobei es sich bei der metabolischen Aktivität um den Metabolismus eines Kohlenhydrats handelt.

9. Reaktionsmedium nach einem der Ansprüche 1 bis 8, das weiterhin ein zweites Substrat für eine enzymatische oder metabolische Aktivität umfasst.

10. Reaktionsmedium nach Anspruch 9, wobei es sich bei dem zweiten Substrat um ein Substrat für eine enzymatische alpha-Glucosidaseaktivität handelt.

11. Reaktionsmedium nach einem der Ansprüche 1 bis 10, wobei das erste und/oder zweite Antibiotikum, das zur Familie der Cephalosporine gehört, aus den folgenden ausgewählt ist:
o einem Cephalosporin der ersten Generation, das vorzugsweise aus Cefalexin, Cefaloridin, Cefalotin, Cefalozin, Cefadroxil, Cefazedon, Cefatrizin, Cefapirin, Cefradin, Cefacetril, Cefrodaxin, Ceftezol ausgewählt ist;
o einem Cephalosporin der zweiten Generation, das vorzugsweise aus Cefoxitin, Cefuroxim, Cefamandol, Cefaclor, Cefotetan, Cefonicid, Cefotiam, Loracarbef, Cefmetazol, Cefprozil, Ceforanid ausgewählt ist;
o einem Cephalosporin der dritten Generation, das vorzugsweise aus Cefotaxim, Ceftazidim, Cefsulodin, Ceftriaxon, Cefmenoxim, Latamoxef, Ceftizoxim, Cefixim, Cefodizim, Cefetamet, Cefpiramid, Cefoperazon, Cefpodoxim, Ceftibuten, Cefdinir, Cefditoren, Ceftriaxon, Cefoperazon, Cefbuperazon ausgewählt ist;
o einem Cephalosporin der vierten Generation, das vorzugsweise aus Cefepim, Cefiprom ausgewählt ist.

12. Reaktionsmedium nach einem der Ansprüche 1 bis 11, wobei das zweite Antibiotikum, das zur Familie der Carbapeneme gehört, aus Meropenem, Ertapenem, Imipenem ausgewählt ist.

13. Reaktionsmedium nach einem der Ansprüche 1 bis 12, wobei die Kombination von zwei Antibiotika aus der Kombination Cefoxitin - Cefotaxim oder Cefoxitin - Ertapenem ausgewählt ist.

14. In-vitro-Verwendung eines Reaktionsmediums nach einem der Ansprüche 1 bis 13 zum Isolieren und Identifizieren von methicillinresistenten *Staphylococcus aureus*-Bakterien (MRSA).

15. Verfahren für den Nachweis und/oder die Identifikation von methicillinresistenten *Staphylococcus* aureus-Bakterien (MRSA) in einer biologischen Probe, bei dem man
a) eine biologische Probe, die methicillinresistente *Staphylococcus aureus*-Bakterien MRSA enthalten kann, auf ein Reaktionsmedium nach einem der Ansprüche 1 bis 13 aufträgt,
b) man dieses inkubiert,
c) man die Kolonien als MRSA-Kolonien identifiziert.

## Claims

1. Reaction medium for detecting and/or identifying Methicillin-resistant *Staphylococcus aureus* (MRSA) bacteria, comprising a predetermined combination of two antibiotics, a first antibiotic which belongs to the cephalosporin family and a second antibiotic, said first and second antibiotics each being at a sub-inhibitory concentration.

2. Reaction medium according to Claim 1, according to which said first antibiotic belongs to the cephamycin subfamily.

3. Reaction medium according to Claim 1 or 2, according to which said second antibiotic belongs to the carbapenem family.

4. Reaction medium according to Claim 1 or 2, according to which said second antibiotic belongs to the cephalosporin family.

5. Reaction medium according to any one of Claims 1 to 4, which also comprises a substrate for detecting an enzymatic or metabolic activity.

6. Reaction medium according to Claim 5, according to which said enzymatic activity is an osidase, esterase or peptidase activity.

7. Reaction medium according to Claim 6, according to which said osidase enzymatic activity is an alpha-glucosidase activity.

8. Reaction medium according to Claim 5, according to which said metabolic activity is the metabolism of a carbohydrate.

9. Reaction medium according to any one of Claims 1 to 8, which also comprises a second substrate for an enzymatic or metabolic activity.

10. Reaction medium according to Claim 9, according to which said a second substrate is a substrate for an alpha-glucosidase enzymatic activity.

11. Reaction medium according to any one of Claims 1 to 10, according to which said first and/or second antibiotic(s) belonging to the cephalosporin family is selected from:
o a first-generation cephalosporin, preferably selected from Cefalexin, Cefaloridine, Cefalotin, Cefazolin, Cefadroxil, Cefazedone, Cefatrizine, Cefapirin, Cefradine, Cefacetrile, Cefrodaxine, Ceftezole;
∘ a second-generation cephalosporin, preferably selected from Cefoxitin, Cefuroxime, Cefamandole, Cefaclor, Cefotetan, Cefonicide, Cefotiam, Loracarbef, Cefmetazole, Cefprozil, Ceforanide;
∘ a third-generation cephalosporin, preferably selected from Cefotaxime, Ceftazidime, Cefsulodine, Ceftriaxone, Cefmenoxime, Latamoxef, Ceftizoxime, Cefixime, Cefodizime, Cefetamet, Cefpiramide, Cefoperazone, Cefpodoxime, Ceftibuten, Cefdinir, Cefditoren, Ceftriaxone, Cefoperazone, Cefbuperazone;
o a fourth-generation cephalosporin, preferably selected from Cefepime, Cefpirome.

12. Reaction medium according to any one of Claims 1 to 11, according to which said second antibiotic belonging to the carbapenem family is selected from Meropenem, Ertapenem and Imipenem.

13. Reaction medium according to any one of Claims 1 to 12, according to which the combination of two antibiotics is selected from the combination Cefoxitin - Cefotaxime or Cefoxitin - Ertapenem.

14. *In vitro* use of a reaction medium according to any one of Claims 1 to 13, for isolating and identifying Methicillin-resistant *Staphylococcus aureus* (MRSA) bacteria.

15. Method for detecting and/or identifying Methicillin-resistant *Staphylococcus aureus* (MRSA) bacteria, in a biological sample, comprising:
a) inocculating the biological sample that may contain Methicillin-resistant *Staphylococcus aureus*, MRSA, bacteria on a reaction medium according to any one of Claims 1 to 13;
b) incubating;
c) identifying the colonies as being MRSA colonies.
